# EUROPEAN PATENT APPLICATION

(11) **EP 2 684 456 A1**
(43) Date of publication of application: **15.01.2014**
(21) Application number: 12757614.8
(22) Date of filing: 05.01.2012
(51) Int. Cl.: A01N 63/04, A01N 31/00

(54) **FUNGICIDAL AND BACTERICIDAL COMPOSITION THAT COMPRISES VOLATILE ORGANIC COMPOUNDS OF NATURAL ORIGIN**

(30) Priority: 11.03.2011 ES 201130341
(71) Applicant: Biofungitek, Sociedad Limitada, 48170 Zamudio (Bizkaia) (ES)
(72) Inventor: UGALDE MARTINEZ, Unai Ona, E-20280 Hondarribia (Gipuzkoa) (ES); RODRIGUEZ URRA, Ana Belen, E-20012 Donostia - San Sebastian (Gipuzkoa) (ES); FUNDAZURI ZUGAZAGA, Olatz, E-48287 Ea (Bizkaia) (ES)
(74) Representative: Durán-Corretjer, S.L.P.
(86) International application number: PCT/ES2012/070002
(87) International publication number: WO 2012/123605

(57) **Abstract**

The present invention relates to a composition with fungicidal and bactericidal activity, which comprises volatile organic compounds (VOCs) obtained from plants and microorganisms, in which said VOCs are C4-C12 fatty alcohols or cyclic alcohols or phenol-derived compounds, or terpene-derived compounds, and also derivatives and mixtures thereof. Furthermore, the present invention likewise relates to the use of said compositions in the protection of agricultural crops, in post-harvest treatments, in food preservation and in the disinfection of facilities and equipment.

## Description

This invention relates to a composition having fungicidal and bactericidal activity comprising volatile organic compounds (VOC) obtained from plants and microoganisms in which the VOC are fatty alcohols of between 4 and 12 carbon atoms such as 2-ethyl-1-hexanol, 3-nonanol, 1-octen-3-ol, hexanol, 3-methyl-1-butanol, isobutanol, 3-octanol, (Z)-3-hexen-1-ol, 1-penten-3-ol, ethanol, isomers, derivatives and mixtures thereof, as well as cyclic alcohols such as menthol or compounds derived from phenols such as phenylethanol, phenylmethanol, 2,4-di-t-butylphenol, isomers, derivatives and mixtures thereof or compounds derived from terpene such as isoborneol, 2-methyl isoborneol, 2-norbonanol, cariophyllene, aristolene, α-bergamotene, naphthalene, α-patchoulene, myrcene, a- and b-phellandrene, limonene, linalool, carvacrol, thymol, camphene, geraniol, nerol, and derivatives and mixtures thereof. This invention also relates to use of these compositions in the protection of agricultural crops, in post-harvesting treatments, in the conservation of foodstuffs and in the disinfection of facilities and equipment.

Fungi and bacteria affecting products after harvesting and foods in general cause considerable economic losses (Prusky, D., Kolattukudy, P.E. (2007) Cross talk between hosts and fungus in post-harvest situations and its effect on symptom development. In Food Mycology: a multifaceted approach to fungi and food. J. Dijksterhuis and R.A. Samson, editors. CRC Press, BocaRaton, 3-25), being responsible for both loss of the commercial value of products and the production of microtoxins which are harmful to humans and animals.

After harvesting it is usual to apply chemicals to control the growth of fungi and bacteria. However, the application of conventional fungicidal and bactericidal compounds gives rise to a series of problems, such as the appearance of resistance (Brent, K.J., Hollomon, D.W. (2007) Fungicide resistance: the assessment of risk. FRAC Monograph No. 1 (2nd edition), Croplife International, Brussels, Belgium) or pesticide residues in foodstuffs, an aspect of legal regulation which is becoming increasingly restrictive. Because of all this there is a need to develop effective products having antifungal and antimicrobial activity with a mode of action involving fast and effective elimination, which are also harmless to human beings and the environment.

The antimicrobial action of soils has been studied for decades. Most natural soils suppress the germination and growth of fungi, a phenomenon known as fungistasis. It is known that the intensity of this fungistasis depends on the physical and chemical properties of the soil and its microbial population (Alabouvette, C. (1999) Fusarium wilt suppressive soils: an example of disease suppressive soils. Australas. Plant Pathol. 28: 57-64 and Toyota K. et al. (1996) Microbiological factors affecting colonisation of soil aggregates by Fusarium oxysporum f. sp. raphani, Soil Biol. Biochem. 28: 1513-1521). Soil microorganisms bring about fungistasis in fundamentally two ways: 1) strong competition for the scarce nutrients in the soil and 2) the presence of antifungal compounds of microbial origin, many of which are volatile organic compounds (VOC) (Insam, H., Seewald, M.S.A., (2010) Volatile organic compounds (VOCs) in soils. Biol. Fertil. Soils. 46: 199-213).

It is known in the art that there are some fungi that produce VOC with antimicrobial properties, such as the fungus *Muscodor albus,* which produces a mixture of VOC lethal to various species of fungi and bacteria that are pathogenic to humans and plants (Strobel, G.A., (2006) Muscodor albus and its biological promise. J. Ind. Microbiol. Biotechnol. 33: 514-522).

Because of their natural origin VOC are very attractive for application to agricultural crops, in post-harvesting treatments, in the preservation of foodstuffs and in the disinfection of facilities and equipment, as they have antifungal and antibacterial activity while being harmless to humans and the environment.

After extensive studies the present authors have surprisingly found that some VOC isolated from cultures of microorganisms that are pathogenic to plants and fruits such as *Botrytis cinerea, Penicillium digitarum, Fusarium oxysporum* or *Mucor racemosus,* or directly isolated from plants and fruits, have activity inhibiting the growth of fungi and bacteria.

Thus one object of this invention is to provide a composition having antifungal and antibacterial activity which comprises a volatile organic compound of natural origin isolated from plants or microorganisms in which the said volatile organic compound is selected from fatty alcohols having between 4 and 12 carbon atoms such as 2-ethyl-1-hexanol, 3-nonanol, 1-octen-3-ol, hexanol, 3-methyl-1-butanol, isobutanol, 3-octanol, (Z)-3-hexen-1-ol, 1-penten-3-ol, ethanol, isomers, derivatives and mixtures thereof, as well as cyclic alcohols such as menthol or compounds derived from phenols such as phenylethanol, phenylmethanol, 2,4-di-t-butylphenol, isomers, derivatives and mixtures thereof or compounds derived from terpene such as isoborneol, 2-methyl isoborneol, 2-norbonanol, cariophyllene, aristolene, α-bergamotene, naphthalene, α-patchoulene, myrcene, a- and b-phellandrene, limonene, linalool, carvacrol, thymol, camphene, geraniol, nerol, and derivatives and mixtures thereof. Furthermore, another object of this invention is to provide use of these compositions in the protection of agricultural crops, in post-harvesting treatments, in the preservation of foodstuffs and in the disinfection of facilities and equipment. The quantity of VOC of natural origin present in the composition according to this invention is at least 2 mg/Lₐᵢᵣ.

One advantage of the composition according to this invention is that because of their nature VOC are volatile at ambient temperature, as a result of which once the product is removed from the facilities in which fungicidal/bactericidal treatment has been carried out, they dissipate quickly and leave virtually no residues on the treated product. The composition according to this invention would therefore preferably be in gaseous form. However this composition may also be in liquid form such as a suspension, dispersion, emulsion, spray or any other type of mixture which remains stable over time or may be incorporated in plastics or natural polymers, waxes or any support in which the composition remains stable over time.

The composition according to this invention falls within the group of contact phytosanitary products, that is the nature of protection against fungal and/or bacteria diseases takes place through contact, as this composition remains on the surface of different parts of the plant or fruit, protecting it on the outside against external attack by fungi and bacteria.

In addition to this, the phytosanitary composition according to this invention can be used as such, or can be used to formulate a product which further comprises different additives used in the art such as surfactants, polymers, alkanising agents, and pH-controlling agents, among many other additives used in the formulation of products used in the agricultural industry.

The fungicidal composition according to this invention may further comprise a fertiliser which may be selected from the group comprising compounds containing nitrogen and/or phosphorus such as urea, melamine, hexamine, dicyanodiamide, ameline, cyanuric acid, melamine nitrate, triethyl phosphite and the like or mixtures thereof.

The composition according to this invention may also comprise any compounds or products having chemical and/or biological activity used in agriculture, such as herbicides, insecticides, plant growth regulators and the like, or mixtures thereof.

This invention is described in greater detail below with reference to various examples. These examples are however not intended to restrict the technical scope of this invention.

### EXAMPLES

Example 1. Identification of volatile organic compounds (VOC) produced by pathogenic fungi.

The VOC were extracted from 96 hour cultures of various fungi pathogenic to plants and fruits, such as *Botrytis cinerea, Penicillium digitarum, Fusarium oxysporum* or *Mucor racemosus,* through solid phase microextraction (SPME). Desorption and identification of the VOC bound to the fibres was achieved through the technique of gas chromatography-mass spectrometry (GC-MS), consulting the Wiley 08 and NIST 05 libraries. The compounds identified particularly in the species *Mucor racemosus* are shown in Table 1.

**Table 1. VOC produced by Mucor racemosus cultured for 96 hours in PDA**

| Retention time (s) | Possible compound |
|---|---|
| 1.1 | Ethanol |
| 2.5 | Propanol |
| 3.88 | Isobutanol |
| 4.8 | Butanol |
| 6.85 | 3-methyl-1-butanol |
| 6.9 | 2-methyl-1-butanol |
| 11.06 | 2,3-butanediol |
| 11.11 | 1-hexanol |
| 14.29 | 1-octen-3-ol |
| 15.58 | 2-ethyl-1-hexanol |
| 16.56 | 1-octanol |
| 17.2 | Phenyl methanol |
| 17.53 | 2-norbornanol |
| 18.47 | Phenyl ethanol |
| 18.5 | Isoborneol |
| 21.35 | α-Bergamotene |

The results obtained from this fungus indicate that there is great coincidence between the various species of fungi studied.

Example 2. Inhibition of growth of the fungus *Botrytis cinerea* and the bacterium *Erwinia carotovora* by VOC of natural origin.

The antifungal and antibacterial activity of the VOC obtained in Example 1 was tested by bioassay in hermetically sealed vials in which the microorganism was cultured while being exposed to the compound under test in the gas phase at different concentrations. The VOC had a purity of more than 95% by weight. The IC₅₀ (50% inhibiting concentration), the MIC (minimum inhibiting concentration) and the MLC (minimum lethal concentration) were calculated.

**Table 2. IC₅₀, MIC and MLG for 2-ethyl-1-hexanol in mg/Lₐᵢᵣ**

| | IC₅₀ | MIC | MLC |
|---|---|---|---|
| *Botrytis cinerea* | 4 | 5 | 10 |
| *Erwinia carotovora* | 15 | 50 | 75 |

**Table 3. IC₅₀, MIC and MLG for hexanol in mg/Lₐᵢᵣ**

| | IC₅₀ | MIC | MLC |
|---|---|---|---|
| *Botrytis cinerea* | 15 | 20 | 40 |
| *Erwinia carotovora* | 150 | >150 | >150 |

**Table 4. IC₅₀, MIC and MLG for phenyl ethanol in mg/Lₐᵢᵣ**

| | IC₅₀ | MIC | MLC |
|---|---|---|---|
| *Botrytis cinerea* | 500 | >1000 | >1000 |
| *Erwinia carotovora* | 1000 | >1000 | >1000 |

**Table 5. IC₅₀, MIC and MLG for isoborneol in mg/Lₐᵢᵣ**

| | IC₅₀ | MIC | MLC |
|---|---|---|---|
| *Botrytis cinerea* | 5 | 40 | 40 |
| *Erwinia carotovora* | >150 | >150 | >150 |

The results obtained through application of the VOC of natural origin according to this invention in the gaseous phase demonstrate that these are effective in inhibiting the growth of pathogenic fungi such as *Botrytis cinerea* and bacteria such as *Erwinia carotovora.* This indicates that the VOC have a wide spectrum of action, and may be effective against the main species of fungi and bacteria which cause damage in the agricultural and food sectors in concentrations as low as 2 mg/Lₐᵢᵣ.

## Claims

1. A composition having antifungal and antibacterial activity comprising as the active ingredient a single volatile organic compound of natural origin isolated from plants or microorganisms in which the single volatile organic compound is a fatty alcohol having between 4 and 12 carbon atoms, or a cyclic alcohol, or a phenol or phenol derivative, or a terpene or terpene derivative.

2. A composition according to claim 1, **characterised in that** the fatty alcohol having between 4 and 12 carbon atoms is selected from the group comprising 2-ethyl-1-hexanol, 3-nonanol, 1-octen-3-ol, hexanol, 3-methyl-1-butanol, isobutanol, 3-octanol, (Z)-3-hexen-1-ol, 1-penten-3-ol, ethanol, isomers, derivatives and mixtures thereof.

3. A composition according to claim 1, **characterised in that** the cyclic alcohol is menthol.

4. A composition according to claim 1, **characterised in that** the phenol or phenol derivative is selected from the group comprising phenyl ethanol, phenyl methanol, 2,4-di-t-butylphenol, their isomers, derivatives and mixtures thereof.

5. A composition according to claim 1, **characterised in that** the terpene or terpene derivative is selected from the group comprising isoborneol, 2-methyl isoborneol, 2-norbonanol, cariophyllene, aristolene, α-bergamotene, naphthalene, α-patchoulene, myrcene, a- and b-phellandrene, limonene, linalool, carvacrol, thymol, camphene, geraniol, and derivatives and mixtures thereof.

6. A composition according to any one of the preceding claims, **characterised in that** the quantity of volatile organic compound in the composition is at least 2 mg/Lₐᵢᵣ·

7. A composition according to any one of the preceding claims, **characterised in that** it further comprises surfactants, polymers, alkanising agents, pH-controlling agents and/or mixtures thereof.

8. A composition according to any one of the preceding claims, **characterised in that** it further comprises a fertiliser selected from the group comprising compounds containing nitrogen and/or phosphorus such as urea, melamine, hexamine, dicyanodiamide, ameline, cyanuric acid, melamine nitrate, triethyl phosphite and the like or mixtures thereof.

9. A composition according to any one of the preceding claims, **characterised in that** the composition is in gaseous or liquid form such as a suspension, dispersion, emulsion, spray or any other type of mixture which remains stable over time or is incorporated in plastic or natural polymers, waxes or any supports in which the composition remains stable over time.

10. A composition according to claim 9, **characterised in that** the composition is in gaseous form.

11. A composition according to any one of the preceding claims, **characterised in that** it further comprises a compound or product having chemical and/or biological activity used in agriculture such as herbicides, insecticides, plant growth regulators and the like, or mixtures thereof.

12. Use of the composition having antifungal and antibacterial activity according to claims 1 to 11, preparation of a formulation of a health product having fungicidal properties.

13. Use of the composition having antifungal and antibacterial activity according to claims 1 to 11 on agricultural crops, in post-harvesting treatments, in the preservation of foodstuffs and in the disinfection of facilities and equipment.
